# EUROPEAN PATENT SPECIFICATION

(11) **EP 0 259 585 B1**
(45) Date of publication and mention of the grant of the patent: **09.06.1993**
(21) Application number: 87110658.9
(22) Date of filing: 23.07.1987
(51) Int. Cl.: C12P 21/08, G01N 33/577, C12N 5/00, C12N 15/00, C07K 15/00

(54) **Monoclonal antibodies specific to surface receptor for IgE and hybrid cell lines producing these antibodies and use thereof**
Monoklonale Antikörper gegen oberflächlichen IgE-Rezeptor, sowie Hybrid-Zellinien zu deren Herstellung und Verwendung
Anticorps monoclonaux spécifiques, contre le récepteur de surface pour IgE ainsi que des lignées cellulaires hybrides produisant de tels anticorps et leur utilisation

(30) Priority: 29.07.1986 EP 86110420; 19.08.1986 EP 86111488
(43) Date of publication of application: 16.03.1988
(73) Proprietor: Kishimoto, Tadamitsu, Prof., 1-3, Yamadaoka Suita Osaka 565 (JP)
(72) Inventor: Kishimoto, Tadamitsu, Prof. Dr., Osaka 584 (JP); Suemura, Masaki, Dr., Osaka 563 (JP); Kikutani, Hitoshi, Dr., Osaka 565 (JP); Barsumian, Edward L., Dr., Osaka 562 (JP)
(74) Representative: Laudien, Dieter, Dr.

(56) References cited:
- CHEMICAL ABSTRACTS, vol. 106, no. 1, January 1987, page 351, abstract no. 3603s, Columbus, Ohio, US; H. KIKUTANI et al.: "Fcepsilon receptor, a specific differentiation marker transiently expressed on mature B cells before isotype switching", & J. EXP. MED. 1986, 164(5), 1455-69
- CHEMICAL ABSTRACTS, vol. 103, no. 11, 16th September 1985, page 162, abstract no. 86103r, Columbus, Ohio, US; E. RECTOR et al.: "Detection and characterization of monoclonal antibodies specific to IgE receptors on human lymphocytes by flow cytometry", & IMMUNOLOGY 1985, 55(3), 481-8
- CHEMICAL ABSTRACTS, vol. 104, no. 15, 14th April 1986, page 556, abstract no. 128114y, Columbus, Ohio, US; & JP-A-60 255 734 (NICHIREI CORP.) 17-12-1985
- CHEMICAL ABSTRACTS, vol. 105, no. 7, 18th August 1986, page 490, abstract no. 59181z, Columbus, Ohio, US; M. CAPRON et al.: "Functional study of a monoclonal antibody to IgEFc repector (FcepsilonR2) of eosinophils, platelets, and macrophages", & J. EXP. MED. 1986, 164(1), 72-89
- CHEMICAL ABSTRACTS, vol. 105, no. 12, September 1986, page 487, abstract no. 95741r, Columbus, Ohio, US; T. NAKAJIMA et al.: "IgE receptors on human lymphocytes. I. Identification of the molecules binding to monoclonal anti-Fce receptor antibodies", & EUR. J. IMMUNOL. 1986, 16(7), 809-14
- BIOLOGICAL ABSTRACTS, vol. 80, no. 11, November 1985, abstract no. 95522, Biological Abstracts, Inc., Philadelphia, P.A., US; E. RECTOR et al.: "Detection and characterization of monoclonal antibodies specific to immunoglobulin E receptors on human lymphocytes by flow cytometry", & IMMUNOLOGY, 55(3), 481-488, 1985
- CHEMICAL ABSTRACTS, vol. 104, no. 3, January 1986, page 379, abstract no. 18371x, Columbus, Ohio, US; S.A. HUDAK et al.: "A microtiter plate assay for detecting IgE-binding molecules and cells bearing Fc receptors for IgE", & J. IMMUNOL. METHODS 1985, 84(1-2), 11-24

## Description

It is known from the literature, although the crucial role of IgE is not yet fully appreciated, that IgE biosynthesis requires the interaction of T and B cells and that the regulation of IgE synthesis has certain peculiarities and involves humoral factors derived from other lymphocytes. It is further known that these factors have affinity to IgE and will bind to precursors of IgE-forming cells, thus enhancing or suppressing their differentiation. In this fashion they play a critical role in IgE-isotype-specific regulation.

Furthermore it is known, that B lymphocytes originate from pluripotent hematopoietic stem cells and differentiate into antibody secreting cells through multistep differentiation stages, such as pre-B cells, immature B cells with surface IgM and mature B cells with surface IgM and IgD. A number of human B cell antigens have been defined by monoclonal antibodies (1-10). However, most B cell specific monoclonal antibodies except for antibodies to plasma cells (5) and activated B cells (9,10) are known to recognize cells of the B lineage at a wide range of differentiation stages from pre-B cells to mature B cells. It has therefore been difficult to identify B cells at a specific differentiation stage by employing such B cell-specific monoclonal antibodies, because in Euro. J. Immunol. 16, 809-814 (1986) are described only monoclonal antibodies which block the IgE-binding activity.

Surprisingly, the antibody designated 3-5 does not show any significant antagonism to the IgE-binding activity, because this antibody recognizes an epitope different from the one recognized by the monoclonal antibodies described in Euro. J. Immunol. 16, 809-814 (1986). These known antibodies recognize the IgE-binding region of the Fc_{ε}-receptor, namely the range of the amino acids 163-282.

An object of the present invention is therefore to provide the new anti-Fc_{ε}R-antibody 3-5 which enable B cells to be identified at a specific differentiation stage as well enabling the different degrees of atopy and Fc_{ε}-receptor expression to be defined as a result by the showing that Fc_{ε}R is a B cell specific phenotype marker, the expression of which is strictly correlated with the differentiation stage of B cells, especially with the isotype switching in B cells. Therefore, Fc_{ε}R positive B cells can be recognized by detection with the new antibody of the expressed Fc_{ε}R, e.g. the expressed Fc_{ε}R in supernatant having a molecular weight of about 25 Kd.

The new anti-Fc_{ε}R-antibody was prepared according to the invention as follows:

### Reagents and antibodies:

Human IgE myeloma protein (PS) was the same preparation as described in reference (6). Human IgG₁ protein was obtained from Osaka Medical Center and Research Institute for Maternal and Child Health (Dr. A. Shimizu). Fluorescein isothiocyanate (FITC)-conjugated goat anti-mouse IgM + IgG antibodies were purchased from Tago, Inc. Burlingame, Ca.; FITC-conjugated anti-Leu 4 was obtained from Becton-Dickinson Monoclonal Center, Inc. Mountain View, Ca.; FITC-anti B1 antibody was obtained from Coulter Immunology, Hialeah, Fl.; the monoclonal anti-B cell antibody, MR11, was prepared as described in reference (12). Avidin-FITC and biotinyl-N-hydroxy succinimide ester were obtained from E.Y. Laboratories, Inc. San Mateo, Ca.; human Igs and monoclonal antibodies were conjugated with biotin or FITC as described in references (13) and (14).

### Human cell lines:

RPMI8866 (8866), an Epstein-Barr virus-transformed B cell line, which is Fc_{ε}R positive, was obtained from John Hopkins University, Md. (Dr. K. Ishizaka). Other B cell lines (CESS, SKW-CL4 and RPMI1788) and T cell lines (CCRF-CEM and MOLT 4) were obtained from Karolinska Institute, Sweden (Dr. G. Klein). The percentage of Fc_{ε}R-bearing cells by rosetting method was 80-95 % in 8866, CESS and SKW-CL4 cells, 50-65 % in RPMI1788 cells, and 0 % in CEM, MOLT 4 and Daudi cells, respectively.

### Somatic cell hybridization and cloning:

BALB/c mice were immunized i.p. with 1 x 10⁷ 8866 cells suspended in Hanks' balanced salt solution. Mice were boosted twice with the same number of cells at 4 weeks intervals. Three hundred million spleen cells were fused with 3 x 10⁷ of hypoxanthine-aminopterine-thymidine (HAT) sensitive P3U1 mouse myeloma cells using polyethylene glycol 4000 (Wako Pure Chemical Inc., Osaka, Japan) as described in reference (15). The cells were suspended in HAT medium (Dulbecco's modified Eagle's medium with HAT, 20 % FCS, 1 % NEAA, antibiotics and glutamine) at 1 x 10⁵ feeder myeloma cells/ml, and 200 µl of the cell suspension were dispensed with BALB/c thymocytes into 96 well tissue culture plates (Falcon 3072, Oxnard, Ca). One half of the culture medium was changed with fresh HAT medium every third day. After 14 days, the HAT medium was replaced with HT and the hybrid clones were grown in regular culture medium after 3-4 weeks. Wells with growing hybrid cells were screened for anti-Fc_{ε}R antibodies, and positive cultures were cloned by limiting dilutions. After testing for anti-Fc_{ε}R antibody activity, selected monoclonal clones were transplanted in BALB/c mice pretreated with pristane (Aldrich Chemical Co., Milwaukee, Wis.) and ascitic fluid was obtained. For this purpose, it may be of advantage to subclone a selected clone according to known methods.

### Selection of hybridomas secreting anti-Fc_{ε}R antibodies by immunofluorescence:

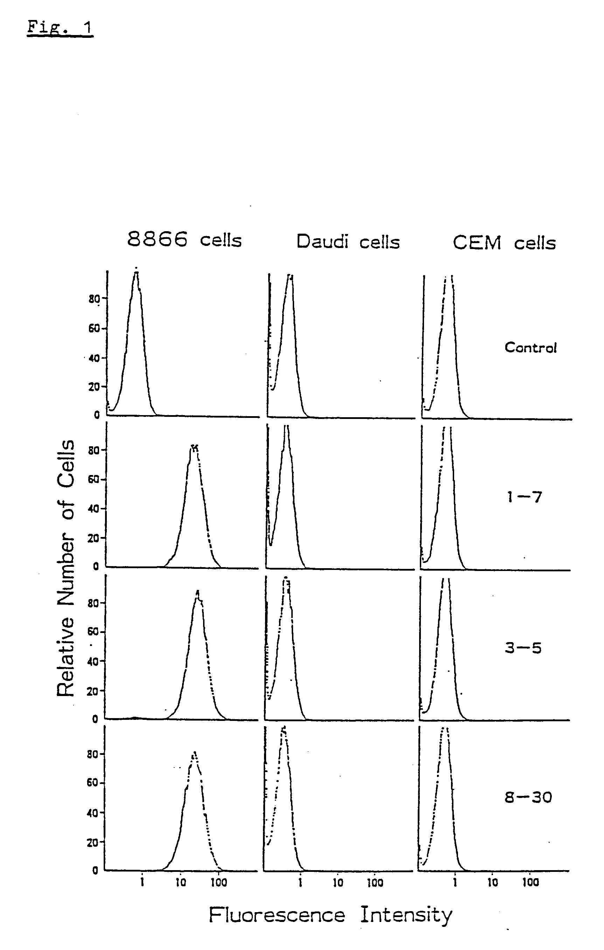

The initial screening of the B hybridomas secreting antiFc_{ε}R antibodies was done on the basis of immunofluorescence with 8866, Daudi, CEM, and Molt 4 cells. A half million of 8866, Daudi, DEM and MOLT 4 cells were incubated with 50 µl of culture supernatants followed by FITC-anti-mouse IgM + IgG and analyzed by a cell sorter (FACS 440, Becton Dickinson, Mountain View, Ca.). After cloning by limiting dilution technique, three hybrid clones, 1-7 (γ2b), 3-5 (γ1) and 8-30 (µ), reacting only with 8866 cells but not with Daudi, CEM, and Molt 4 cells, were selected as possible candidates for anti-Fc_{ε}R antibodies (Fig 1.). A thus selected clone can be sub-cloned according to known methods e.g. by the limiting dilution technique, thus for example when sub-cloning clone 8-30 a clone was obtained designated as clone 8-248-12 secreting the same antibody as clone 8-30.

In order to confirm whether these monoclonal antibodies reacted only with Fc_{ε}R-positive cell lines, several T and B cell lines were screened by FACS analysis. It was observed that these monoclonal antibodies reacted with 8866, 1788, CESS and SKW-CL 4 cells, which express Fc_{ε}R, but not with Daudi cells nor with the T cell lines, CEM and Molt 4, which are negative for Fc_{ε}R, indicating that these monoclonal antibodies may be reacting with Fc_{ε}R but not with other cell surface determinants such as FcγR or Ia antigens.

### Selection of hybridomas secreting antibodies with inhibitory activity on IgE-rosette formation:

Fc_{ε}R on lymphocytes were detected by an assay employing fixed ox erythrocytes (ORBC) coated with human IgE (11) and the percentage of IgE-rosette-forming cells (RFC) was estimated after subtracting the percentage of non-specific RFC binding with fixed ORBC coated with BSA. For IgE-rosette inhibition assay, 25 µl of 8866 cells (5 x 10⁶/ml) were mixed with 100 µl hybridoma culture supernatants or control medium and incubated for 1 hr at 4°C. After incubation, 25 µl of 2 % modified ORBC coated with IgE or BSA was added, and the mixture was centrifuged at 200g for 8 min followed by incubation for 1 hr at 4°C. IgG rosette inhibition assay was performed essentially in the same manner employing Daudi cells and ORBC sensitized with IgG fraction of rabbit antiORBC antiserum.

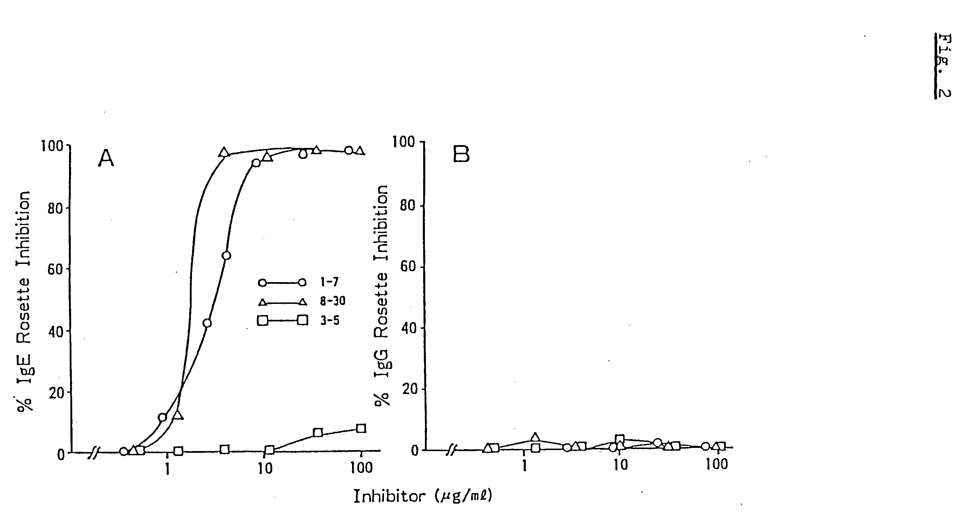

As shown in Fig 2, two monoclonal antibodies, 1-7 and 8-30, inhibited the rosette formation of 8866 cells with IgE-coated ox red cells (ORBC) up to 97 % when used at concentrations of 5 to 10 µg/ml, while the other antibody, 3-5, showed marginal inhibitory activity only at high concentrations (100 µg/ml). The two monoclonal antibodies (1-7 and 8-30) did not inhibit the rosette formation of Daudi cells with IgG-ORBC, even when 100 µg/ml of antibodies were employed. These results prove that the monoclonal antibodies, 1-7 and 8-30, recognize Fc_{ε}R, but 3-5 may recognize a different epitope of Fc_{ε}R or molecules distinct from Fc_{ε}R.

### Antibody purification:

Hybridoma clones, 3-5 (γ1), 8-30 (µ), and 1-7 (γ2b) were selected as the possible candidates for anti-Fc_{ε}R antibodies by means of their inhibitory effect on IgE-rosette formation. The first two of which were deposited at the Collection nationale de micro-organisms, Institut Pasteur (C.N.C.M), 25, rue du Dr. Roux, 75724 Paris, Cedex 15 on the 24th July 1986, under the file numbers I-583 and I-584 respectively, and, under the form of Budapest Treaty at the National Collection of Animal Cell Cultures, PHLS Centre for Applied Microbiology and Research, Porton Down, Salisbury, Wiltshire SP4 OJG, United Kingdom, on April 28, 1987 under the file numbers ECACC 87042801 and ECACC 8704802 respectively. The necessary monoclonal antibodies were purified from ascites by 50 % saturated ammonium sulfate precipitation followed by gel filtration using Sepharose 6B (Pharmacia Fine Chemicals, Uppsala, Sweden) for IgM class or by ion exchange chromatography using QAE-Sephadex (Pharmacia Fine Chemicals) for IgG₁ and IgG2b classes.

### Preparation of human mononuclear cells (MNC):

Peripheral blood mononuclear cells were obtained from normal donors and atopic patients with bronchial asthma or atopic dermatitis but not undergoing systemic corticosteroid therapy. Serum IgE levels of these patients were between 1,000-15,000 IU/ml. Tonsillar mononuclear cells were obtained from patients with chronic tonsillitis. MNC were prepared by Ficoll-Hypaque gradient centrifugation, and B cell-enriched populations (E⁻ cells) were prepared by rosetting with (2-aminoethyl)-isothiouronium bromide (AET, Aldrich Chemical Co.) -treated sheep red blood cells.

### Indirect and direct immunofluorescence analysis:

One million cells were incubated with 0,1 µg to 1 µg of biotin-conjugated monoclonal antibody for 20 min in the first step, washed several times with staining buffer (RPMI1640 minus biotin, riboflavin and phenol red/2 % FCS/10mM HEPES/0,1 % NaN₃), incubated with Texas Red (TR)-avidin for 20 min in the second step and then washed three times with staining buffer. Direct staining of cells was performed by incubating 1 x 10⁶ cells with FITC-conjugated antibody followed by washing three times. Propidium iodide (PI, 10 µg/ml) was included in the last five minutes of the second step to label the dead cells. The one-color and twocolor immunofluorescence analyses by FACS 440 and VAX computer is described in reference (12).

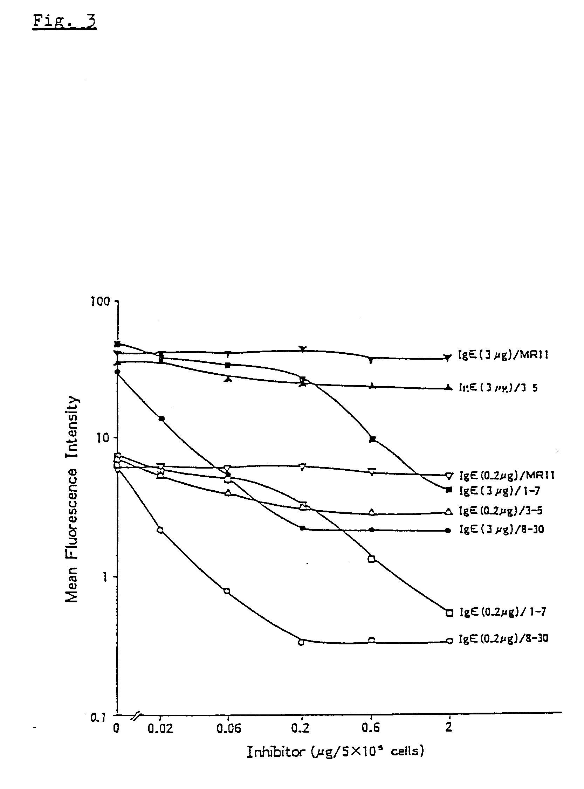

As shown in Fig. 3, both 1-7 and 8-30 monoclonal antibodies, as expected, blocked the IgE binding to 8866 cells in a dose-dependent fashion. The monoclonal antibody, 8-30, was much more effective than 1-7. This may be due to the fact that 8-30 is of IgM isotype. On the other hand, the monoclonal antibody, 3-5, could display only a slight inhibition of IgE binding at high concentrations. The control monoclonal antibody, MR11 of IgM class, did not affect the binding of IgE to 8866 cells.

### Inhibition assay in FACS analysis:

One half million of peripheral MNC, precultered for 1 hr and washed three times to remove cytophilic IgG or IgE, were exposed to various concentrations of monoclonal antibodies in 20 µl of staining buffer in 96 well microtiter plates at 4°C for 30 min. After incubation, cells were washed twice with staining buffer, incubated with FITC-anti Bl and biotinated IgGl or IgE for 20 min, and washed twice. Subsequently, the cells were incubated with TR-avidin, washed and analyzed by FACS.

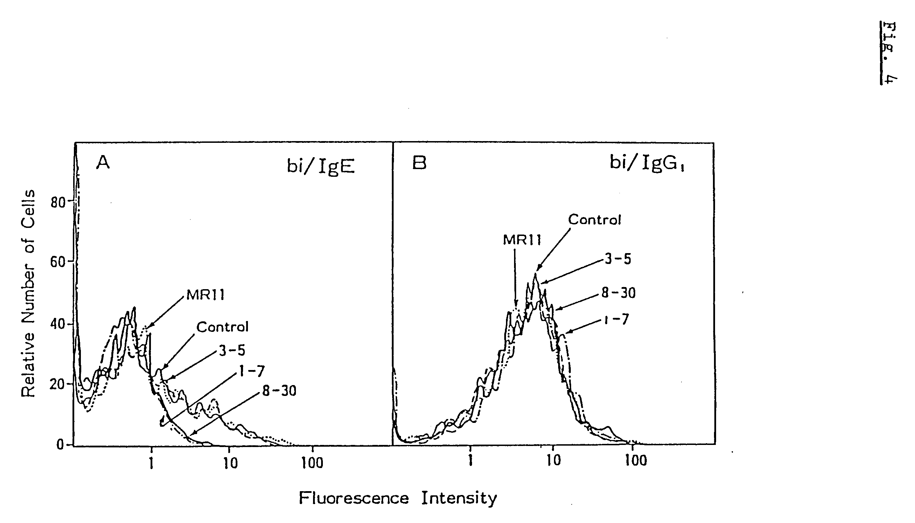

As shown in Fig 4, the binding of IgE to Bl-positive cells was inhibited by the presence of either 1 µg/5 x 10⁵ cells of 1-7 or 8-30 antibody, but not by the monoclonal antibody, 3-5 or the control antibody, MR11. The same concentration of the monoclonal antibodies did not inhibit IgG binding to Bl-positive cells. These results again demonstrated that the two monoclonal antibodies, 1-7 and 8-30, recognize FC_{ε}R and the monoclonal antibody, 3-5, might be directed to a different epitope of Fc_{ε}R or to a different molecule closely associated with the receptor.

### Analysis of the determinants recognized by these monoclonal antibodies:

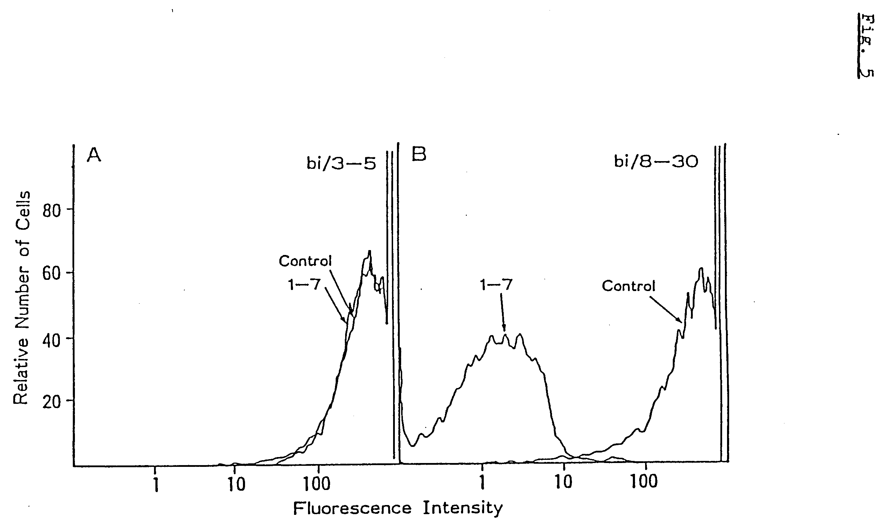

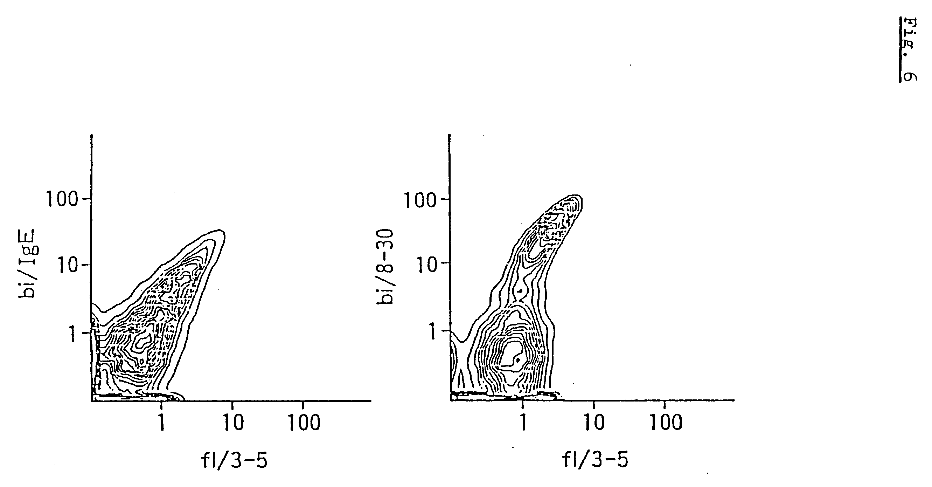

Cross staining utilizing different monoclonal antibody conjugates was employed in order to establish a correlation in the recognition of Fc_{ε}R epitopes defined by these antibodies. 8866 cells were stained with biotin-3-5 or -8-30 followed by TR-avidin in the presence or absence of the monoclonal antibody 1-7. As shown in Fig 5, the monoclonal antibody, 1-7, could inhibit the binding of 8-30 to 8866 cells (Fig 5-B) but not the binding of 3-5 (Fig 5-A). These results demonstrate that the two monoclonal antibodies, 1-7 and 8-30, recognize the same epitope of Fc_{ε}R. In order to study whether the monoclonal antibody, 3-5, recognized a different epitope of Fc_{ε}R or a molecule distinct from Fc_{ε}R, two color FACS analysis of tonsillar B cells with the monoclonal antibodies, 8-30 and 3-5, or IgE and 3-5, was carried out. Tonsillar B cells were stained with FITC-conjugated 3-5 antibody and biotin-conjugated 8-30 antibody or IgE followed by TR-avidin. The expression of the antigen recognized by the two monoclonal antibodies, 8-30 and 3-5 or the expression of Fc_{ε}R and the antigen recognized by 3-5 was perfectly correlated (Fig 6), suggesting that the two monoclonal antibodies, 8-30 and 3-5, recognized different epitopes on the same Fc_{ε}R molecules.

### Molecules recognized by these monoclonal antibodies:

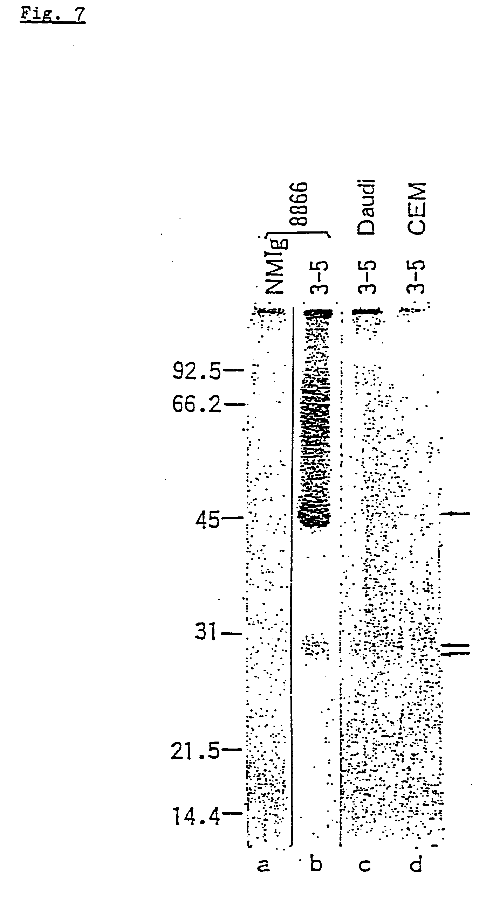

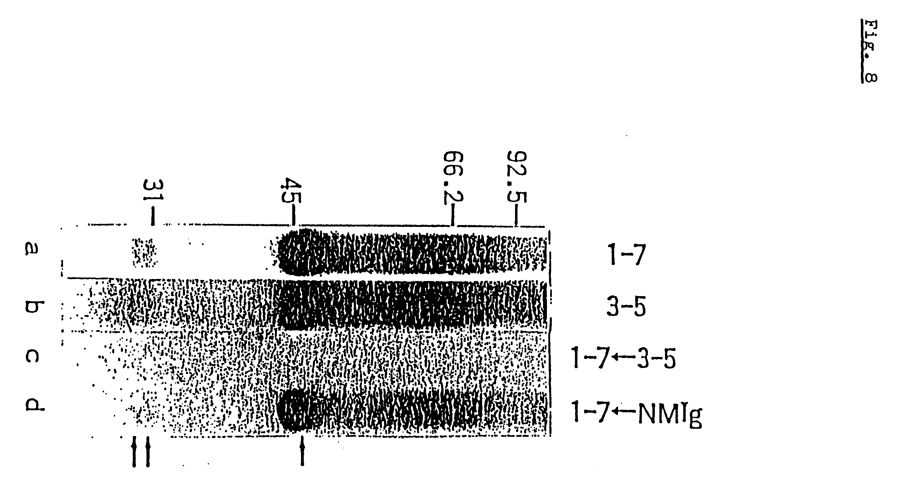

In order to analyze the molecules recognized by these monoclonal antibodies, Fc_{ε}R-positive 8866 and Fc_{ε}R-negative Daudi and CEM cells were surface labelled with ¹²⁵I and lysed in lysis buffer containing 0,5 % NP-40. After pre-absorption with HSA-Sepharose, lysates were immunoprecipitated with the monoclonal antibody, 1-7 or 3-5, or normal mouse Ig (NMIg), and analyzed by SDS-PAGE (Fig 7 and Fig 8). In the lysates of 8866 cells, one major band with the molecular weight of 46 Kd and one or two minor bands with the molecular weight of 25-29 Kd as well as broad smear between 60 to 90 Kd were detected under reducing and non-reducing conditions, whereas lysates from Daudi cells or CEM cells did not show any bands with the monoclonal antibodies. In order to study whether molecules recognized by two monoclonal antibodies, 1-7 and 3-5, were identical or not, sequential immunoprecipitation was carried out. Cell lysates of ¹²⁵I-labelled 8866 cells were immunoprecipitated with either 3-5 monoclonal antibody or NMIg, and effluents were further immunoprecipitated with the monoclonal antibody, 1-7. The analysis by SDS-PAGE is shown in Fig 8. Both monoclonal antibodies, 1-7 and 3-5, precipitated 46 Kd molecules (Fig. 8 lane a and b). Preclearing with the monoclonal antibody, 3-5, virtually eliminated the molecules recognized by the monoclonal antibody, 1-7, (Fig 8 lane c) but preclearing with NMIg did not affect the reactivity of the monoclonal antibody, 1-7, (Fig 8 lane d) indicating that molecules recognized by the two monoclonal antibodies, 1-7 and 3-5, were identical. The results demonstrate that all the three monoclonal antibodies, 1-7, 3-5 and 8-30, recognize Fc_{ε}R on human B lymphocytes.

In order to qualitatively and quantitatively estimate Fc_{ε}R as antigen using the new antibodies the standard immunoassay techniques can be used.

These techniques are based on the formation of a complex between the antigenic substance to be determined and one or more antibodies in which complex one or more may be "marked", whereby the qualitative and/or quantitative estimation of the antigens after the separation of the marked, complexed antigens or antibodies is made possible.

In the case of a competitive immunoassay technique the antigenic substance is investigated in a liquid test sample in competition with a known amount of a marked antigen for a limited amount of antibody binding sites. Accordingly the amount of the marked antigen which is bound to the antibody is inversely proportional to the amount of the antigen under investigation in the test sample.

In immunometric methods on the other hand marked antibodies are used, and in such assays the amount of marked antibody bound to the complex is proportional to the amount of antigenic substance in the liquid sample.

Immunometric assays are especially suitable for estimation of polyvalent "antigens" in antigenic substances which are capable of forming complexes with two or more antibodies simultaneously. Typically in such assays a quantity of unmarked antibody is bound to a solid carrier which is insoluble in the liquid sample under investigation and a quantity of soluble, marked antibody is used so that the qualitative and/or quantitative determination of the amount of the terniary complex formed between the solid-phase-antibody, the antigen and the marked antibody is rendered possible.

In the latter method the antibody bound to the solid phase is first contacted with the sample under investigation, in order that the antigen in the sample forms a binary solid-phase-antibody:antigen complex. After a suitable incubation period the solid carrier is washed so as to remove the sample liquid including any unreacted antigen, and then brought into contact with a solution containing a known quantity of marked antibody.

After a second incubation period during which the marked antibody is enabled to form a complex with the antigen which is bound to the carrier via the unmarked antibody the carrier is washed a second time, to remove unreacted, marked antibody. In a simple "Yes-No" assay to confirm whether an antigen is or is not present in the test sample, the above mentioned second-time washed carrier can be investigated. The amount of confirmed marked antibody can be compared with a control sample which is free from antigen. The confirmation of marked antibody in an amount which is considerably greater than the background level (as established by the sample) then indicates the presence of antigen. Quantitative analysis is possible by comparing results with results from calibrated samples containing known amounts of antigen. This type of analysis is frequently referred to as a "Sandwich-assay" because the antigen binds two antibodies at different binding sites.

In the above mentioned assays the usual carriers are glass, polystyrene, polypropylene, polyethylene, dextran, nylon, amylase, natural or chemically modified cellulose, polyacrylamide, agarose or magnetite;
as marker there may be used enzymes, radioisotopes, metallchelates or fluorescent, chemiluminescent or bioluminescent compounds;
as enzymes, for example malatdehydrogenase, staphyloccalnuclease, delta-5-steroidisomerase, α-glycerine-phosphatdehydrogenase, triosephosphateisomerase, horseradish-peroxidase, alkaline-phosphatase, asparaginase, glucoseoxidase, β-galactosidase, ribonuclease, glucoamylase or acetylcholinesterase;
as radioisotopes ³H, ¹²⁵J, ¹²⁷J, ³²P, ³⁵S and ¹⁴C;
as fluorescent compounds fluorescein-isothiocyanate, rhodamine, phycoerythrin, phycocyanin, allophycocyanin, o-phthalaldehyde or fluorescamin;
as chemiluminescent compounds luminol, isoluminol, an aromatic acridinium ester, imidazol, an acridinium salt or an oxalic acid ester; and
as a bioluminescent compounds luciferin, luciferase or aquorin.

Moreover, an antibody in accordance with the present invention may be bound to a low molecular weight hapten for example biotin, dinitrophenyl, pyridoxal or fluorescamin. This hapten can then be recognized through a further, specific reaction, for example in the case of biotin using avidin, or fluorescamin using a specific anti-hapten-antibody.

In addition, the activity of an enzyme used as a marker can be used to strengthen the signal which is to be measured.

It is especially preferred to use horseradish-peroxidase as marker as this enzyme is capable of reacting with numerous substrates. Besides, this molecule is relatively small and can very easily bind with an antibody, for example using the periodate method.

For the qualitative or quantitative determination of Fc_{ε}R, when the Fc_{ε}R is radioactively marked, it is preferred to use the competetive radio immunoassay (RIA) in which especially polyclonal antibody preparations or antibody serums are employed; when the antibody is radioactively marked it is preferred to use the immunoradiometric assay (IRMA); and the enzyme-linked immunoadsorbent assay (ELISA) when the antibody is marked with an enzyme. The Elisa according to the invention comprises usually a solid phase carrying a monoclonal antibody to said surface receptor for IgE, e.g. a carrier coated with monoclonal antibody 3-5 and/or 8-30, and a second a monoclonal antibody to said surface receptor for IgE bound to a detectable label such as a radioactive isotope, an enzyme, a fluorescent or chemiluminiscent compound or a second antibody specifically binding to an above mentioned antibody and carrying any one of the afore-mentioned labels, e.g. alkaline phosphatase-conjugated anti-mouse IgM, preferably alkaline phosphatase-conjugated anti-mouse IgM.

The monoclonal antibody 3-5 is of γ₁ class, does not significantly inhibit the binding of IgE, and binds to a specific epitope of Fc_{ε}R.

The monoclonal antibody 8-30 is of µ class and inhibits the binding of IgE and binds to a specific epitope of Fc_{ε}R.

Therefore, in the new ELISA the above mentioned monoclonal antibodies bind in combination selectively to Fc_{ε}R found in culture supernatants or lysates of RPMI8866 cells, EB-transformed cells and serum derived from atopic and non-atopic individuals. The double antibody sandwich technique utilizing the monoclonal antibodies 3-5 and 8-30 identifies the presence of Fc_{ε}R in serum from atopic and non-atopic individuals and enables the state of hypersensitivity of an individual to be followed. This technique is a potential diagnostic tool in allergy since, as in the case of determining the level of IgE in serum, it allows monitoring and diagnosis of an allergic condition.

For example, the ELISA may be carried out as follows:
Ninety-six well microtiter plates are coated with monoclonal antibody 3-5 in coating buffer, 100 µl of 10 µg/ml. The plates are incubated overnight at 4°C, and washed with rinse buffer 4 times before use. 100 µl of test sample is added to each well and incubated for 2 hours at room temperature (the test sample can be diluted first with diluent buffer to allow a titration). The test samples are dumped and the plate washed 4 times with rinse buffer followed by the addition of 100 µl of second monoclonal antibody, 8-30 diluted to 1 µg/ml in diluent buffer and incubated for 2 hours at room temperature. The wells are washed 4 times with rinse buffer followed by the addition of 100 µl of goat anti-mouse IgM conjugated to alkaline phosphatase (pretitrated) and incubated for 2 hours at room temperature. The wells are washed with rinse buffer and 100 µl of 1 mg/ml of substrate, p-nitrophenyl phosphate disodium added. The enzymatic reaction is allowed to proceed and the optical (color) determination measured at 405 and 620 nm.

Using as second antibody, an antibody from a sub-clone, designated here as antibody 8-248-12, the procedure can be as follows:
The coated microtiter plates (3-5, 10 µg/ml, 4°C, overnight) were washed four times with rinse buffer.

A serial dilution of RPMI 8866 cell supernatant from 10 U/ml to 0.1 U/ml and test samples in appropriate dilutions, e.g. yeast fermentation supernatants in diluent buffer (1:2 - 1:64) were added to the plates.

After incubation for two hours at room temperature the plates were washed and incubated with the second antibody 8-248-12 (1 µg/ml) for two hours.

The plates were washed and then incubated with a goat anti-mouse IgM conjugated to alkaline phosphatase or horseradish peroxidase (HRP) for two hours.

After washing the plates four times the enzyme reaction was started by adding 100 µl of 1 mg/ml of p-nitrophenyl phosphate disodium (or 100 µl of substrate solution: 2.88 mg/ml o-phenylene diamine (OPD) and 1.25 mg/ml sodium perborate in 0.1 M citrate buffer: aqua dest. = 2:1 for HRP conjugate).

After incubation for an appropriate time in the dark, the optical density was measured at 405 and 620 nm (or 492 and 405 nm for OPD).

A standard curve can be constructed from the serial dilution of the RPMI supernatant. The Fc_{ε}R-concentration of the samples were read from the generated curve.

### References

1. Kishimoto, T., and K. Ishizaka. 1973. Regulation of antibody response in vitro. VI. Carrier-specific helper cells for IgG and IgE antibody response. J. Immunol., 111:720.
2. Kishimoto, T., Y. Hirai, M. Suemura, and Y. Yamarua. 1976. Regulation of antibody response in different immunoglobulin classes. I. Selective suppression of anti-DNP IgE antibody response by preadministration of DNP-coupled mycobacterium. J. Immunol., 117:396.
3. Kishimoto, T. 1982. IgE class-specific suppressor T cells and regulation of the IgE response. Prog. Allergy., 32:265.
4. Ishizaka, K. 1984. Regulation of IgE synthesis. Ann. Rev. Immunol., 2:159.
5. Katz, D.H. 1985. The IgE antibody system is coordinately regulated by FcR epsilon-positive lymphoid cells and IgE-selective soluble factors. Int. Archs Allergy appl. Immunol., 77:21.
6. Deguchi, H., M. Suemura, A. Ishizaka, Y. Ozaki, s. Kishimoto, Y. Yamamura, and T. Kishimoto. 1983. IgE class-specific suppressor T cells and factors in humans. J. Immunol., 131:2751.
7. Suemura, M., A. Ishizaka, S. Kobatake, K. Sugimura, K. Maeda, K. Nakanishi, S. Kishimoto, Y. Yamamura, and T. Kishimoto. 1983. Inhibition of IgE production in B hybridomas by IgE class-specific suppressor factor from T hybridomas. J. Immunol., 130:1056.
8. Suemura, M., J. Yodoi, M. Hirashima, and K. Ishizaka. 1980. Regulatory role of IgE-binding factors from rat T lymphocytes. I. Mechanism of enhancement of IgE response by IgE-potentiating factor. J. Immunol., 125:148.
9. Hirashima, M., J. Yodoi, and K. Ishizaka. 1980. Regulatory role of IgE-binding factor from rat T lymphocytes. III. IgE specific suppressive factor with IgE-binding activity. J. Immunol., 125:1442.
10. Uede, T., T.F. Huff, and K. Ishizaka. 1984. Suppression of IgE suhthesis in mouse plasma cells and B cells by rat IgE-suppressive factor. J. Immunol., 133:803.
11. Gonzalez-Molina, A., and H.L. Spiegelberg. 1977. A sub-population of normal human peripheral B lymphocytes that bind IgE. J. Clin. Invest., 59:616.
12. Kikutani, H., T. Kimura, J. Nakamura, R. Sato, A. Muraguchi, N. Kawamura, R.R. Hardy, and T. Kishimoto. 1986. Expression and function of an early activation marker restricted to human B cells. J. Immunol., in press
13. Goding, J. 1976. Conjugation of antibodies with fluorochromes. Modification to standard method. J. Immunol. Methods., 13:215.
14. Bayer, E.A., and M. Wilcheck. 1978. The avidin-biotin complex as a tool in molecular biology. Trends Biochem. Sci., 3:N257.
15. Maruyama, S., T. Naito, K. Kakita, S. Kishimoto, Y. Yamamura, and T. Kishimoto. 1983. Preparation of a monoclonal antibody against human monocyte lineage. J. Clin. Immunol., 3:57.

### Legends for Figures

### Fig. 1

FACS analysis of 8866, Daudi and CEM cells with monoclonal, anti-Fc_{ε}R antibodies (1-7, 3-5 and 8-30). Cells were incubated with culture supernatants of the hybrid clones followed by staining with FITC-goat-anti-mouse IgM + IgG antibodies and applied to FACS analysis.

### Fig. 2

Inhibitory activity of the monoclonal anti-Fc_{ε}R antibodies on IgE (panel A) and IgG (panel B) rosette formation. For IgE-rosette assay, 8866 cells were preincubated with varying concentrations of the monoclonal antibodies or control medium and subsequently incubated with fixed ORBC coated with IgE or BSA. IgG-rosette assay was carried out employing Daudi cells and rabbit IgG-coated ORBC.

### Fig. 3

Blocking of IgE binding to 8866 cells by the monoclonal antibodies. 8866 cells were stained with either 3 µg or 0.2 µg/5x10⁵ cells of biotin-IgE followed by TR-avidin in the presence or absence of varying concentrations of the monoclonal antibodies (1-7, 3-5 and 8-30) or a B cells-specific monoclonal antibody (MR11) and applied to FACS analysis. The mean fluorescence intensity was calculated by VAX-11 computer.

### Fig. 4

Blocking of IgE binding but not IgG binding to peripheral MNC by the monoclonal antibodies. Peripheral MNC were preincubated with either 1 µ/5x10⁵ cells of the anti-Fc_{ε}R monoclonal antibodies or a B cell specific antibody (MR11). After washing, cells were incubated with FITC-anti-Bl and 3 µg/5x10⁵ cells of biotinated IgE or IgG₁ followed by TR-avidin and analyzed by FACS. Relative number of IgE (panel A) or IgG (panel B) binding cells to Bl positive cells (B cells) was shown in the histograms.

### Fig. 5

Inhibition of the binding of the monoclonal antibody 8-30 but not 3-5 to 8866 cells by the monoclonal antibody, 1-7. 8866 cells were stained with 1 µg/1x10⁶ cells of biotin-3-5(A) or 0.1 µg/1x10⁶ cells of biotin-8-30(B) followed by TR-avidin in the presence or absence of 9 µg/1x10⁶ cells of 1-7 and analysed by FACS.

### Fig. 6

Correlated expression of Fc_{ε}R and the antigen recognized by the monoclonal antibody, 3-5 or 8-30. Tonsillar B cells were stained with 0,5 µg/1x10⁶ cells of FITC-labelled 3-5 and 0.1 µg/1x10⁶ cells of biotin-labelled 8-30 or 3 µg/1x10⁶ cells of biotinated IgE followed by TR-avidin and the two color FACS analysis was carried out.

### Fig. 7

SDS-PAGE analysis of the surface molecules reactive with the monoclonal antibody (3-5). 8866 (lane a and b), Daudi (lane c) and CEM (lane d) were surface-labelled with ¹²⁵I and cell lysates were immunoprecipitated with either the monoclonal antibody (3-5) (lane b through d) or NMIg (lane a). The precipitates were analysed by SDS-PAGE under non-reducing condition. (Identical results were obtained under reducing condition).

### Fig. 8

Sequential immunoprecipitation of the surface molecules of 8866 cells with the monoclonal antibodies 3-5 and 1-7. 8866 cells were surface-labelled with ¹²⁵I and cell lysates were immunoprecipitated with 1-7 (lane a) or 3-5 (lane b). After precipitation with 3-5 or NMIg, the supernatant was precipitated with 1-7 (lane c and d) and analysed by SDS-PAGE.

## Claims

1. A process for the preparation of a hybridoma capable of producing an antibody which is of type γ1, recognizes a surface receptor for IgE (Fc_{ε}R) on human B lymphocytes, but does not significantly inhibit the binding of IgE to the Fc_{ε}R, which comprises immunising an animal with a source of IgE receptors, fusing spleen cells from said immunised animal with non-immunoglobulin secreting myeloma cells, and selecting from the resulting hybridomas a cell line which produces a monoclonal antibody having the desired binding specificity and, if desired, subsequently sub-cloning said hybridoma.

2. A process as claimed in claim 1, wherein B lymphoblastoid cells producing Fc_{ε}R are used as the source of IgE receptors and the immunised animal is a mouse.

3. A process as claimed in claim 2, wherein RPMI 8866 cells are used as B lymphoblastoid cells.

4. A process as claimed in any of claims 1 to 3 wherein P3UI mouse myeloma cells are used.

5. A monoclonal antibody, designated 3-5, obtainable from the hybridoma deposited at the ECACC under 87042801, wherein said antibody is of type γ1, recognizes a surface receptor for IgE (Fc_{ε}R) on human B lymphocytes, but does not significantly inhibit the binding of IgE to the Fc_{ε}R.

6. A monoclonal antibody as claimed in claim 5 wherein said Fc_{ε}R are expressed on RMPI 8866, RMPI 1788, CESS and SKW-C14 cells.

7. A monoclonal antibody as claimed in claims 5 and 6 wherein said antibody recognizes a major component of the receptor having a molecular weight of about 46 Kd and a minor component of the receptor having a molecular weight of about 24 to 29 Kd.

8. A monoclonal antibody as claimed in any of claims 5 to 7, wherein said antibody is capable of precipitating a major band of about 46 Kd and a minor band of about 24 to 29 Kd from a lysate of Fc_{ε}R-positive RPMI 8866 cells.

9. A monoclonal antibody as claimed in any one of claims 5 to 8 wherein said antibody is bound to a solid carrier or to a detectable label.

10. A monoclonal antibody as claimed in claim 9 wherein said antibody is bound to a detectable label comprising an enzyme, a radioisotope, a metallochelate, a fluorescent, a chemiluminescent or a bioluminescent compound or a low molecular weight hapten.

11. A monoclonal antibody as claimed in claim 10 wherein said antibody is bound to biotin or horseradish-peroxidase.

12. An immunoassay method for the determination of IgE receptors in a biological material which comprises
(a) incubating a sample of said biological material with a monoclonal antibody as claimed in any of claims 5 to 11; and
(b) determining the amount of said antibody bound to said receptors or remaining unbound.

13. An immunoassay method as claimed in claim 12 wherein said biological material is blood or serum.

14. An immunoassay method as claimed in claim 12 or in claim 13 wherein said monoclonal antibody is bound to biotin and determination of bound or unbound antibody is carried out using Texas-Red-avidin and dual laser FACS.

15. An immunoassay method as claimed in any of claims 12 to 14 which comprises
(a) incubating a biological sample with a first antibody as claimed in claim 5 and a second antibody, one of said first and second antibodies being bound to a solid carrier and the other being bound to a detectable label; and
(b) determining the amount of ternary complex formed between IgE receptors and first and second antibodies.

16. A kit useful for the detection of B cells expressing surface receptors for IgE comprising
(a) a solid phase carrying a monoclonal antibody as claimed in any of claims 5 to 8; and
(b) a monoclonal antibody as claimed in claim 10 or 11.

17. The hybridoma deposited under ECACC 87042801 or a subclone thereof.

18. A process for the preparation of a monoclonal antibody as claimed in any of claims 5 to 11 which comprises culturing a hybridoma as claimed in claim 17, recovering the monoclonal antibody so produced and, if desired, subsequently binding said antibody to a solid carrier or a detectable label.

## Patentansprüche

1. Verfahren zur Herstellung eines Hybridoms, welches befähigt ist einen Antikörper zu produzieren, der vom Typ γ1 ist und einen Oberflächenrezeptor für IgE (Fc_{ε}R) auf menschlischen B-Lymphozyten erkennt, aber kaum die Bindung des IgE mit dem Fc_{ε}R hemmt, dadurch gekennzeichnet, daß ein Tier mit einer Quelle der IgE Rezeptoren immunisiert wird, die Milzzellen von einem so immunisierten Tier mit Myelomzellen, die kein Immunoglobulin ausscheiden, fusioniert werden und von den so erhaltenen Hybridomen eine Zellinie ausgewählt wird, die einen monoklonalen Antikörper mit der gewünschten Bindungsspezifität produziert, und gewünschtenfalls anschließend das so erhaltene Hybridom subkloniert wird.

2. Verfahren gemäß Anspruch 1, dadurch gekennzeichnet, daß Fc_{ε}R produzierende B lymphoblastoide Zellen als Quelle für den IgE-Rezeptor und als zu immunisierendes Tier eine Maus benützt wird.

3. Verfahren gemäß Anspruch 2, dadurch gekennzeichnet, daß als B lymphoblastoide Zellen RPMI8866-Zellen benützt werden.

4. Verfahren beansprucht in irgendeinem Anspruch der Ansprüche 1 bis 3, dadurch gekennzeichnet, daß P3UI Mäuse-Myelom-Zellen verwendet werden.

5. Ein als 3-5 bezeichneter monoklonaler Antikörper, erhältlich von dem bei der ECACC unter 88704281 hinterlegtem Hybridom, wobei dieser Antikörper vom Typ γ1 ist und einen Oberflächenrezeptor für IgE (Fc_{ε}R) auf menschlichen B Lymphocyten erkennt, aber kaum die Bindung von IgE mit dem Fc_{ε}R bindet.

6. Ein monoklonaler Antikörper gemäß Anspruch 5, dadurch gekennzeichnet, daß dieser Fc_{ε}R auf RMPI8866-, RMPI1788-, CESS- und SKW-C14-Zellen exprimiert wird.

7. Ein monoklonaler Antikörper gemäß den Ansprüchen 5 und 6, dadurch gekennzeichnet, daß dieser Antikörper eine größere Komponente des Rezeptors, die ein Molekulargewicht von ungefähr 46 Kd aufweist, und eine kleinere Komponente des Rezeptors, die ein Molekulargewicht von ungefähr 24 bis 29 Kd aufweist, erkennt.

8. Ein monoklonaler Antikörper beansprucht in irgendeinem der Ansprüche 5 bis 7, dadurch gekennzeichnet, daß dieser Antikörper fähig ist, von einem Fc_{ε}R-positiven RPMI8866-Zell-Lysat eine größere Komponente von ungefähr 46 Kd und eine kleinere Komponente von ungeführ 24 bis 29 Kd zu fällen.

9. Ein monoklonaler Antikörper beansprucht in irgendeinem der Ansprüche 5 bis 7 dadurch gekennzeichnet, daß diese Antikörper an einen festen Träger oder an ein detektierbares Merkmal gebunden ist.

10. Ein monoklonaler Antikörper gemäß Anspruch 9, dadurch gekennzeichnet, daß als detektierbares Merkmal ein Enzym, ein Radioisotop, ein Metallchelat, eine fluoreszierende, chemieluminascierende oder bioluminescierende Verbindung oder ein niedermolekulares Hapten verwendet wird.

11. Ein monoklonaler Antikörper gemäß Anspruch 10, dadurch gekennzeichnet, daß dieser Antikörper an Biotin- oder Meerrettich-Peroxidase gebunden ist.

12. Ein Immunoassay-Verfahren zur Bestimmung von IgE-Rezeptoren in einem biologischen Material, dadurch gekennzeichnet, daß
(a) eine Probe dieses biologischen Materials mit einem monoclonalen Antikörper nach einem der Ansprüche 5 bis 11 inkubiert wird und
(b) der von diesem Rezeptor ungebundene oder gebundene Teil dieses Antikörpers bestimmt wird.

13. Ein Immunoassay-Verfahren gemäß Anspruch 12, dadurch gekennzeichnet, daß als biologisches Material Blut oder Serum verwendet wird.

14. Eine Immunoassay-Methode gemäß Anspruch 12 oder 13, dadurch gekennzeichnet, daß dieser Antikörper an Biotin gebunden ist und die Bestimmung des gebundenen oder ungebundenen Antikörpers mittels Texas-Red-Avidin oder mittels Dual-Laser-TACS erfolgt.

15. Eine Immunoassay-Methode nach einem der Ansprüche 12 bis 14, dadurch gekennzeichnet, daß
(a) eine biologische Probe mit einem ersten Antikörper gemäß Anspruch 5 und einem 2. Antikörper, wobei einer dieser Antikörper an einen festen Träger und der andere an ein detektierbares Merkmal gebunden ist, inkubiert wird und
(b) der Dreier-Komplex, der von der IgE-Rezeptoren, dem ersten und zweiten Antikörper gebildet ist, bestimmt wird.

16. Ein Kit zur Bestimmung von durch B-Zellen exprimierten Oberflächen-Rezeptoren für IgE, dadurch gekennzeichnet, daß dieser
(a) aus einer festen Phase, die einen monoklonalen Antikörper nach einem der Ansprüche 5 bis 8 trägt, und
(b) aus einem monoklonalen Antikörper gemäß Anspruch 10 oder 11 besteht.

17. Das Hybridom hinterlegt unter der ECACC 87042801 oder dessen Subclon.

18. Verfahren zur Herstellung eines monoklonalen Antikörpers nach einem der Ansprüche 5 bis 11, dadurch gekennzeichnet, daß ein Hybridom gemäß Anspruch 17 kultiviert, der so gebildete Antikörper isoliert und gewünschtenfalls anschließend dieser Antikörper an einen festen Träger oder an ein detektierbares Merkmal gebunden wird.

## Revendications

1. Procédé pour la préparation d'un hybridome capable de produire un anticorps qui est du type γ1 et reconnaît le récepteur de surface pour IgE (Fc_{ε}R) sur des lymphocytes B humains sans toutefois inhiber de façon significative la liaison d'IgE sur Fc_{ε}R, qui comprend l'opération consistant à immuniser un animal avec une source de récepteurs IgE, à faire fusionner les cellules de la rate de l'animal immunisé avec des cellules de myélome sécrétant la non-immunoglobuline, et à choisir à partir des hybridomes obtenus une lignée cellulaire qui produit un anticorps monoclonal ayant la spécificité de liaison souhaitée et, si on le désire, à sous-cloner ultérieurement cet hybridome.

2. Procédé selon la revendication 1, dans lequel les cellules de lymphoblastoïde B produisant Fc_{ε}R sont utilisées en tant que source de récepteurs d'IgE et l'animal immunisé est une souris.

3. Procédé selon la revendication 2, dans lequel des cellules RMPI 8866 sont utilisées comme cellules de lymphoblastoïde B.

4. Procédé selon l'une quelconque des revendications 1 à 3, dans lequel on utilise des cellules de myélome de souris P3UI.

5. Anticorps monoclonal, désigné 3-5, pouvant être obtenu à partir de l'hybridome déposé auprès de l'ECACC sous la référence 87042801, dans lequel l'anticorps est du type γ1 et reconnaît un récepteur de surface pour IgE (Fc_{ε}R) sur des lymphocytes B humains, sans toutefois inhiber de façon significative la liaison d'IgE sur Fc_{ε}R.

6. Anticorps monoclonal selon la revendication 5, dans lequel Fc_{ε}R sont exprimés sur des cellules RMPI 8866, RMPI 1788, CESS et SKW-C14.

7. Anticorps monoclonal selon les revendications 5 et 6, dans lequel l'anticorps reconnaît un composant principal du récepteur ayant un poids moléculaire d'environ 46 Kd et un composant mineur du récepteur ayant un poids moléculaire d'environ 24 à 29 Kd.

8. Anticorps monoclonal selon l'une quelconque des revendications 5 à 7, dans lequel l'anticorps est capable de faire précipiter une bande principale d'environ 46 Kd et une bande mineure d'environ 24 à 29 Kd à partir d'un lysat de cellules positives RMPI 8866-Fc_{ε}R.

9. Anticorps monoclonal selon l'une quelconque des revendications 5 à 8, dans lequel l'anticorps est lié à un porteur solide ou à un marqueur détectable.

10. Anticorps monoclonal selon la revendication 9, dans lequel l'anticorps est lié à un marqueur détectable comprenant une enzyme, un radioisotope, un métallochélate, un composé fluorescent, un composé chimioluminescent ou bioluminescent ou un haptène à faible poids moléculaire.

11. Anticorps monoclonal selon la revendication 10, dans lequel l'anticorps est lié à la biotine ou à un peroxydase de raifort.

12. Procédé de test immunologique pour la détermination de récepteurs IgE dans un matériau biologique qui comprend les étapes consistant à
(a) incuber un échantillon du matériau biologique avec un anticorps monoclonal selon l'une quelconque des revendications 5 à 11 ; et
(b) déterminer la quantité de l'anticorps lié aux récepteurs ou restant non lié.

13. Procédé de test immunologique selon la revendication 12, dans lequel le matériau biologique est du sang ou un sérum.

14. Procédé de test immunologique selon la revendication 12 ou la revendication 13, dans lequel l'anticorps monoclonal est lié à la biotine et la détermination de l'anticorps lié ou non lié s'effectue en utilisant l'avidine Texas-Red et FACS double laser.

15. Procédé de test immunologique selon l'une quelconque des revendications 12 à 14 qui comprend les opérations consistant à
(a) incuber un échantillon biologique avec un premier anticorps selon la revendication 5 et avec un second anticorps, l'un des premier et second anticorps étant lié à un porteur solide et l'autre étant lié à un marqueur détectable ; et
(b) déterminer la quantité de complexe ternaire formé entre les récepteurs IgE et les premier et second anticorps.

16. Nécessaire utile à la détection de cellules B exprimant des récepteurs de surface pour IgE comprenant
(a) une phase solide portant un anticorps monoclonal selon l'une des revendications 5 à 8 ; et
(b) un anticorps monoclonal selon la revendication 10 ou 11.

17. Hybridome déposé sous la référence ECACC 87042801 ou son sous-clone.

18. Procédé pour la préparation d'un anticorps monoclonal selon l'une quelconque des revendications 5 à 11 qui comprend les opérations consistant à mettre en culture un hydridome selon la revendication 17, à récupérer l'anticorps monoclonal ainsi produit et, si on le souhaite, à lier ultérieurement l'anticorps à un porteur solide ou à un marqueur détectable.
